# EUROPEAN PATENT APPLICATION

(11) **EP 3 599 287 A1**
(43) Date of publication of application: **29.01.2020**
(21) Application number: 18460046.8
(22) Date of filing: 23.07.2018
(51) Int. Cl.: C12Q 1/6883

(54) **PRIMERS FOR DETECTING THE CAUSATIVE MUTATION IN HORSES PREDISPOSING TO DEVELOPMENT OF HYPERKALEMIC PERIODIC PARALYSIS AND USE OF THE SAME**

(71) Applicant: Centrum Badan DNA Spolka z Ograniczona Odpowiedzialnoscia, 61-132 Poznan (PL)
(72) Inventor: Antosik, Pawel, 61-132 Poznan (PL); Lisiak-Teodorczyk, Karolina, 61-132 Poznan (PL); Wojciechowicz, Jacek, 61-132 Poznan (PL)
(74) Representative: Malewska, Ewa

(57) **Abstract**

The invention discloses primers for amplification by PCR of a *SCN4A* gene fragment, including variant M_001081761.1: c.4248C>G, being the causative mutation contributing to development of hyperkalemic periodic paralysis in horses, which primers constitute a pair of: forward primer - HYPP-F and reverse primer - HYPP-R, having the following sequences:
Primer F (HYPP-F): TATGACTTTGTGACGAAGCAGGT
Primer R (HYPP-R): CCACAATGGACAGGATGACAAC
where the symbols A, C, G, T have their usual meaning and define individual nucleotides.

The primers according to the invention satisfy the following basic criteria: they are 23 and 22 bases long (F = 23 bases, R = 22 bases, respectively), their melting temperature is in the range of 60-63°C and their ratio of GC to AT bases is suitable for oligonucleotides that are primers for the DNA sequence amplification.

The invention also relates to the use of the primers as defined above in a PCR reaction for amplification of the *SCN4A* gene sequence comprising the site of the causative mutation contributing to development of hyperkalemic periodic paralysis in horses.

According to the invention, a kit for carrying the genetic test for the presence of mutations in the *SCN4A* gene in horses, which includes information on conditions of the PCR reaction and sequencing of the amplified DNA fragments by the Sanger's method, primers and components of the reaction mixture, is characterized in that it comprise the HYPP-F and HYPP-R primers as defined above.

<110>  CENTRUM BADAN DNA Spolka z ograniczona odpowiedzialnoscia.

<120>  PRIMERS FOR DETECTING THE CAUSATIVE MUTATION IN HORSES
            PREDISPOSING TO DEVELOPMENT OF HYPERKALEMIC PERIODIC
            PARALYSIS AND USE OF THE SAME
            (Startery do wykrywania mutacji sprawczej u koni
predysponuj¹cej
            do zachorowañ na hiperkaliemiczne pora¿enie okresowe oraz ich
            zastosowanie)

<130>  I.215200.ep

<140>  not known yet
<141>  2018-07-23

<160>  2

<170>  PatentIn version 3.3



<210>  1
<211>  20
<212>  primer 1 (forward)

<400>  1
TATGACTTTGTGACGAAGCAGGT          23



<210>  2
<211>  20
<212>  primer 2 (reverse)

<400>  2
CCACAATGGACAGGATGACAAC          22

## Description

The field of technology to which the present invention belongs is the field of molecular diagnostics in horses. The invention relates to new primers for genotyping of the causative mutation predisposing to hyperkalemic periodic paralysis (HYPP) and to the implementation of a methodology of detection of the mutation in genetic tests identifying in horses a predisposition to development of hyperkalemic periodic paralysis.

The present invention is of a crucial importance for diagnostics of hyperkalemic periodic paralysis in horses. Hyperkalemic periodic paralysis - HYPP, in horses, is a disease with a genetic basis, caused by autosomal mutation of a dominant character, concerning the gene responsible for the proper functioning of cellular sodium channels. HYPP is one of the common diseases of the muscular system in horses. It manifests itself in a significant weakness, uncontrollable tremor and muscle contraction as well as cardiac arrhythmia. The disease can manifest itself at various stages of animals' life. The symptoms occur temporarily, and their severity may range from barely noticeable complaints through frequent and severe attacks of muscle weakness, up to death. The disease is often associated with respiratory failure (Brosnahan M.M., Brooks S.A., Antczak D.F.: Equine clinical genomics: A clinician's primer. Equine Vet. J. 2010, 42: 658-670).

Hyperkalemic periodic paralysis is inherited in autosomal-dominant mode, one allele with a mutation is enough to allow symptoms of the disease to be developed in an individual. The causative mutation for HYPP in horses is c.4248C>G, located in the 23rd exon of the *SCN4A* gene (i.e. sodium voltage-gated channel alpha subunit 4 gene). The gene is located in horses in chromosome 11 (ECA 11), and its length is 27700 bp. *SCN4A* encodes the alpha subunit of the skeletal muscle sodium channel - length of 1834 amino acids (molecular weight 207487 Da). As a result of the described change in the protein product, leucine occurs at the phenylalanine site at position 1416 (p.Phe1416Leu), which changes the spatial structure of the channel. This disturbs the restoration of the membrane resting potential after its initial depolarization, due to the temporary loss of the ion transfer capability of the sodium-potassium pump. Under the circumstances, the level of sodium ions in the cells increases and the level of potassium ions in the blood is temporarily critically high. It results in a constant depolarization of myocytes through their hyperexcitability, which leads to excessive contractions and muscle tremors, as well as transient paralysis attacks.

Heterozygotes have a milder course of HYPP. However, homozygotes often manifest the symptoms already within a few days after birth. Among the latter also a higher mortality was observed (Brosnahan M.M., Brooks S.A., Antczak D.F.: Equine clinical genomics: A clinician's primer. Equine Vet. J. 2010, 42: 658-670 and Naylor J.M.: Equine hyperkalemic periodic paralysis: Review and implications. Can. Vet. J. 1994, 35: 279-285). So far, the disease is incurable, but the rapid introduction of veterinary supervision can significantly reduce its severity. In such cases, early animal diagnostics plays an important role, since it enables fast introduction of appropriate veterinary care. Undoubtedly, genetic tests for the presence of mutations in the *SCN4A* gene offer the most effective diagnostic method (Lengele J.P., Belge H., Devuyst O.: Periodic paralyses: when channels go wrong. Nephrol. Dial. Transplant. 2007, 23:1098-1101).

The aim of the invention is to provide instruments for improving HYPP diagnosis in horses, including primers, i.e. specific fragments of the sequence, for amplification of the *SCN4A* gene fragment by PCR, including the variant studied M_001081761.1: c.4248C>G and use of the primers and also the kit for genetic test for presence of the mutation in the *SCN4A* gene in horses.

The above objectives have been achieved by developing a solution according to the invention.

The invention covers a pair of primers for amplifying by PCR the *SCN4A* gene fragment comprising variant M_001081761.1: c.4248C>G, constituting a causative mutation contributing to development of hyperkalemic periodic paralysis in horses, which primers constitute a pair of: 1. forward primer F - primer HYPP-F and 2. reverse primer R - primer HYPP-R, having the following sequences:
1. Primerr F (HYPP-F): TATGACTTTGTGACGAAGCAGGT
2. Primer R (HYPP-R): CCACAATGGACAGGATGACAAC
wherein the symbols A, C, G, T have their usual meaning and define individual nucleotides.

Primers according to the invention satisfy the following basic criteria:
▪ have length of 23 and 22 bases (respectively F = 23 bases, R = 22 bases),
▪ their melting point is in the range of 60-63°C,
▪ their proportion of GC to AT bases is appropriate for oligonucleotides that are primers for the amplification of DNA sequences.

The invention also relates to the use of the above-defined primers in a PCR reaction for amplification of the *SCN4A* gene sequence comprising the site of the causative mutation predisposing to the development of hyperkalemic periodic paralysis in horses.

The invention covers also a kit for carrying the genetic test for presence of mutations in the *SCN4A* gene in horses, including the HYPP-F and HYPP-R primers of the invention.

The primers of the invention are characterized by complementarity to a particular fragment of the DNA sequence comprising the c.4248C>G mutation and in the PCR environment enable amplification of this fragment, thus making possible detection of the change sought.

In combination with the Sanger's sequencing technology, the solution according to the invention reduces the cost of a single mutation analysis and significantly increases the speed and sensitivity of the analysis.

The invention is described below with reference to the attached drawing, in which:

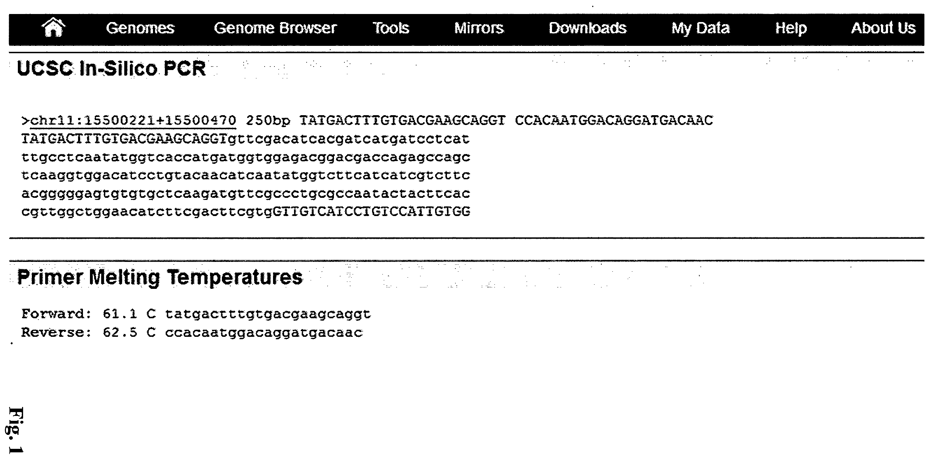

- Fig. 1: shows a fragment of the *SCN4A* gene being amplified by PCR using the HYPP-F and HYPP-R primers of the invention; the sequences to which the primers according to the invention are complementarily attached are indicated by capital letters.

### Detailed description of the invention

Basic information about the main aspects of the present invention is presented below.

In the course of work on the present invention, the latest techniques for developing primers of the invention and a genetic test for determining the presence of a mutant gene encoding for the sodium-potassium alpha subunit, using the HYPP-F and HYPP-R primers of the invention were used, thus significantly improving the genotyping and identification of the given mutation.

The present invention allows to conduct an analysis of the sequence of the *SCN4A* gene alleles responsible for the occurrence of the mutation causing hyperkalemic periodic paralysis in horses. Primers of the invention for the amplification of the *SCN4A* gene sequence allow identification of a variant encoding the α-channel of the skeletal muscle sodium channel, the whose homozygous and heterozygous mutations cause hyperkalemic periodic paralysis in horses. It is important to detect the mutations early because, as it is mentioned above, in homozygotes the HYPP symptoms can often be observed already within a few days after birth, and among them that the mortality rate is higher. Although heterozygotes can be expected to have a milder course of HYPP, early identification of the risk should allow for minimization of the adverse effects of the disease.

The primers of the invention find their use in a genetic test allowing for genotyping of c.4248C>G mutation in the *SCN4A* gene, which test involves three stages of the laboratory procedure:
▪ PCR - polymerase chain reaction,
▪ the Sanger's sequencing
▪ analysis of the results.

The kit of the invention for carrying the genetic test for detecting the presence of the mutations in the *SCN4A* gene in horses, comprises the HYPP-F and HYPP-R primers of the invention and an indication of PCR conditions and sequencing of its products.

### PCR reaction

The primers of the invention are used to amplify the precisely defined DNA regions comprising the *SCN4A* gene. In the PCR reaction following are used: the DNA the fragment of which is to be amplified, a mixture of nucleotides, thermostable DNA polymerase and primers specifically designed to correspond to the change under investigation, i.e. short DNA fragments complementary to the fragments of the amplified gene template.

There are two types of primers: Primer F (forward), the sequence of which is the same as the sequence to be multiplicated and Primer R (reverse), the sequence of which is complementary to the multiplicated one. In this way, they form a pair of primers suitable for amplification of the precisely defined regions of the template DNA.

Amplification is carried out separately for each analyzed sample and it consists of a cycle of repeated three reaction steps: denaturation, annealing and elongation, in other words a synthesis of a new DNA chain. Each one of the three steps takes place at a precisely defined temperature that depends on the primers used, the reagents included in the reaction mixture and the amplified DNA fragment.

The presence of amplicons is then confirmed by agarose gel electrophoresis in the presence of appropriate DNA length markers, which additionally enable verification of the length of the PCR products obtained.

Carrying the PCR reaction out requires a proper selection of parameters: appropriate reaction conditions, primers for the amplification reaction and selection of components of the reaction mixture. As it results from the above description, to conduct the PCR reaction, it is not necessary to know the sequence of the tested gene, just the knowledge of the nucleotide sequence in the sections flanking the examined change is sufficient.

The most important criteria to be met by the primers according to the invention, that allow their use for the genotyping of the *SCN4A* gene, are: length of the primers ranging from 20 to 30 bases, melting points between 61-63°C and the appropriate GC to AT bases ratio.

Genetic research based on the Sanger's sequencing technology provides for a huge advance in medicine. The studies ensure quick and accurate diagnostics, which in turn allow for effective prophylaxis and treatment.

Sequencing of amplified DNA fragments by the Sanger's method is the second stage of laboratory testing. The method involves a controlled termination of DNA chain synthesis. The method is based on PCR reaction, wherein in the reaction mixture in addition to the deoxyribonucleotide triphosphates (dATP, dCTP, dGTP, dTTP) there are fluorescently labeled dideoxyribonucleotide triphosphates (ddATP, ddCTP, ddGTP, ddTTP). These are nucleotides that do not contain the 3' hydroxyl group of ribose and thus prevent the formation of phosphodiester bonds and further chain elongation. Dideoxynucleoside triphosphates are therefore inhibitors of DNA synthesis. In the result, a pool of molecules is obtained, which molecules vary in length and are terminated with one of the four dideoxynucleosides, identifying a given nucleotide - adenine, thymine, guanine or cytosine.

The Sanger's sequencing technology has several advantages in terms of the solution according to the invention, among others:
▪ the present genetic test for the determination of the *SNC4A* gene mutation allows identification of the same in a single analysis,
▪ accuracy of the test performed is higher than 99.9% for a single base, i.e. it is incomparably higher than in other laboratory methods,
▪ the present test allows for the analysis of the most important region of the analyzed gene, thus guaranteeing the highest diagnostic effectiveness,
▪ the genetic test is non-invasive and painless - it is enough to collect a swab or a peripheral blood, the latter being a preferred biological material for carrying out the test according to the invention.

The following working example provides a basic information on practical use of the present solution.

### Example 1.

A sample of a horse biological material was collected. The DNA was isolated from the material by an inorganic method. The quality and purity of the isolated genetic material was determined by electrophoresis and by spectrophotometric method.

Amplification of the exon 23 fragment in which the *SNC4A* gene is located was carried out using PCR - polymerase chain reaction. The pair of the above described primers was used:
1. Primer HYPP-F (forward) of the sequence: 5' TATGACTTTGTGACGAAGCAGGT 3' and
2. Primer HYPP-R (reverse) of the sequence: 5' CCACAATGGACAGGATGACAAC 3'.

The length of the amplified DNA fragment is 250 bp and the fragment includes a sequence fragment ranging from the 4030 nucleotide to the 4279 nucleotide (inclusive). Details are summarized in Table 1.

**Table 1. The length of amplicons for the pair of primers used to analyze the mutation c.4248C>G of the SCN4A gene, and their properties.**

| Gene | Sequence of the primer from 5 'to 3' | Tm ° C | GC% | Estimated length (pz) of the product |
|---|---|---|---|---|
| *SCN4A* | TATGACTTTGTGACGAAGCAGGT | 61,1 | 42,31 | 250 |
| | CCACAATGGACAGGATGACAAC | 62,5 | 41,67 | |

As the last stage of laboratory tests, sequencing of the amplified DNA fragments by the Sanger's method was performed. Sequencing was performed on the ABI PRISM 3100 sequencer (Applied Biosystems).

For sequencing, purified DNA fragments of the *SNC4A* gene, amplified by means of PCR, were used as templates. For the preparation of sequential reactions by a chain termination method, the BigDye ™ Terminator v3.1 Cycle Sequencing Kit (Applied Biosystems) was used.

In order to determine the sequence of the analyzed DNA, a polyacrylamide gel electrophoresis was performed by capillary electrophoresis. The results were obtained in the form of chromatograms generated by the sequencer's software and then analyzed using *Sequence Scanner Software v1.0* (Applied Biosystems).

The sequences of the primers used, the composition of the reaction mixture and the conditions of the sequential reactions are presented in Tables 2 and 3, respectively.

**Table 2. Composition of the sequencing reaction mixture**

| | Initial concentration | Volume in 1 run (µl) |
|---|---|---|
| Purified PCR product | 30 ng/µl | 2 |
| Water | | 2 |
| Primer (HYPP-F or HYPP-R) | 5 µM | 2 |
| Premix reaction mixture | 1x | 4 |
| Total | | 10 |

**Table 3. Sequencing reaction conditions**

| | Temperature (°C) | | Time |
|---|---|---|---|
| Initial denaturation | 95 | | 2 min. |
| Denaturation | 95 | 25 cycles | 20 sec. |
| Annealing | 50 | | 15 sec. |
| DNA synthesis | 60 | | 1 min. |
| Storage | 4 | | untill switching-off |

After the sequencing reaction, the products obtained were again purified by ethanol precipitation in the presence of ammonium acetate. The purified sequencing reaction product was suspended in 10 µl of a loading solution comprising formamide having denaturing nucleic acids properties. In order to determine the sequence of the analyzed DNA, an electrophoretic separation was performed by capillary polyacrylamide gel electrophoresis method.

### Analysis of the results

The results were obtained in the form of chromatograms generated by the sequencer's software. The files were then exported and analyzed using *Sequence Scanner Software v1.0* (Applied Biosystems), which allowed the obtained nucleotide sequence reading.

### References

1. Brosnahan M.M., Brooks S.A., Antczak D.F.: Equine clinical genomics: A clinician's primer. Equine Vet. J. 2010, 42: 658-670;
2. Naylor J.M.: Equine hyperkalemic periodic paralysis: Review and implications. Can. Vet. J. 1994, 35: 279-285;
3. Lengele J.P., Belge H., Devuyst O.: Periodic paralyses: when channels go wrong. Nephrol. Dial. Transplant. 2007, 23:1098-1101;
4. Zabek T., Bugno-Poniewierska M., Gurgul A.: Defekty genów u koni czystej krwi arabskiej - problemy hodowli. Rocz. Nauk Zootech. 2013, 40:127-132;
5. Naylor J.M.: Equine hyperkalemic periodic paralysis: Review and implications. Can. Vet. J. 1994, 35: 279-285;
6. UniProt Database, https://www.uniprot.org/, update: 01.2018.
7. Naylor J.M.: Equine hyperkalemic periodic paralysis: Review and implications. Can. Vet. J. 1994, 35: 279-285;
8. Annandale E.J., Valberg S.J., Mickelson J.R., Seaquist E.R.: Insulin sensitivity and skeletal muscle glucose transport in horses with equine polysaccharide storage myopathy. Neuromuscul. Disord. 2004, 13:666-674;
9. Mccue M.E., Valberg S.J., Jackson M., Borgia L., Lucio M., Mickelson J.R.: Polysaccharide storage myopathy phenotype in quarter horse-related breeds is modified by the presence of an RYR1 mutation. Neuromuscul. Disord. 2009; 19: 37-43;
10. Scott E.Y., Penedo M.C.T., Murray J.D., Finno C.J.: Defining Trends in Global Gene Expression in Arabian Horses with Cerebellar Abiotrophy. The Cerebellum. 2017, 16: 462-472;
11. Brault L.S., Famula T.R., Penedo M.C.T.: Inheritance of cerebellar abiotrophy in Arabians. Am. J. Vet. Res. 2011, 72: 940-944;
12. Brault L.S., Cooper C.A., Famula T.R., Murray J.D., Penedo M.C.T.: Mapping of equine cerebellar abiotrophy to ECA2 and identification of a potential causative mutation affecting expression of MUTYH. Genomics. 2011, 97: 121-129.

## Claims

1. Primers for amplification by PCR of the *SCN4A* gene fragment including the variant M_001081761.1: c.4248C>G being the causative mutation contributing to development of hyperkalemic periodic paralysis in horses, which primers constitute a pair of a forward primer - HYPP-F and a reverse primer - HYPP-R, having the following sequences:
Primer F (HYPP-F): TATGACTTTGTGACGAAGCAGGT
Primer R (HYPP-R): CCACAATGGACAGGATGACAAC
where the symbols A, C, G, T have their usual meaning and define individual nucleotides.

2. Primers according to claim 1, which meet the following basic criteria: they are 23 and 22 bases long (F = 23 bases, R = 22 bases, respectively), their melting temperature is in the range of 60-63°C and their ratio of GC to AT bases is suitable for oligonucleotides that are primers for the DNA sequence amplification

3. The use of the primers as claimed in claim 1 to 2, in a PCR reaction to amplify the *SCN4A* gene sequence comprising the site of the causative mutation contributing to development of hyperkalemic periodic paralysis in horses.

4. A kit for carrying the genetic test for the presence of mutations in the *SCN4A* gene in horses, which includes information on conditions of the PCR reaction and sequencing of the amplified DNA fragments by the Sanger's method, primers and components of the reaction mixture, **characterized in that** it comprise the HYPP-F and HYPP-R primers as defined above in claims 1-2.
